Europäisches Patentamt

European Patent Office

Office européen des brevets

(11) Publication number: **0 213 536 B1**

(12) **EUROPEAN PATENT SPECIFICATION**

(45) Date of publication of patent specification: **18.03.92**

(51) Int. Cl.⁵: **C12P 13/08**, //(C12P13/08, C12R1:01,1:19)

(21) Application number: **86111424.7**

(22) Date of filing: **19.08.86**

(54) **Process for producing L-threonine by fermentation.**

(30) Priority: **23.08.85 JP 183925/85**
**27.02.86 JP 42581/86**

(43) Date of publication of application:
**11.03.87 Bulletin 87/11**

(45) Publication of the grant of the patent:
**18.03.92 Bulletin 92/12**

(84) Designated Contracting States:
**DE FR GB IT**

(56) References cited:
**DE-A- 2 044 907**
**US-A- 3 375 173**

**CHEMICAL ABSTRACTS, vol. 80, no. 11, 18th March 1974, page 242, abstract no. 58409h, Columbus, Ohio, US; & JP-A-73 77 090 (KANEGAFUCHI CHEMICAL INDUSTRY CO., LTD) 17-10-1973**

(73) Proprietor: **TORAY INDUSTRIES, INC.**
**2, Nihonbashi-Muromachi 2-chome Chuo-ku Tokyo 103(JP)**

(72) Inventor: **Yamada, Katsushige**
**115-6, Nakanogo Kita Nishiharu-cho**
**Nishi-Kasugai-gun Aichi-ken, 481(JP)**
Inventor: **Tsutsui, Hiromi I-305, Toray Narumi Shataku**
**1-81, Ikegami-dai Midori-ku**
**Nagoya-shi Aichi-ken, 458(JP)**
Inventor: **Yotsumoto, Kyousuke 2, Toray Wakakusa**
**Shataku 5, Wakakusa-cho Minami-ku**
**Nagoya-shi Aichi-ken, 457(JP)**
Inventor: **Takeuchi, Masae**
**16, Tenjin Kitano-cho**
**Konan-shi Aichi-ken, 483(JP)**
Inventor: **Shirai, Makoto**
**20-7, Yutakadai Misaki-cho**
**Toyoake-shi Aichi-ken, 470-11(JP)**

(74) Representative: **Kador & Partner**
**Corneliusstrasse 15**
**W-8000 München 5(DE)**

EP 0 213 536 B1

CHEMICAL ABSTRACTS, vol. 80, no. 17, 29th April 1974, page 177, abstract no. 93041d, Columbus, Ohio, US; HIRAKAWA, TAMOTSU et al.: "Microbial production of L-threonine. III. Mechanism of L-threonine production in Escherichia coli auxotrophs", & AGR. BIOL. CHEM. 1974, 38(1), 77-84

**Description**

Background of the invention

(a) Field of the Invention

This invention relates to a process for producing L-threonine by fermentation.

(b) Prior Art

There hitherto has been known that the microorganism belonging to the genus Providencia or Proteus of which the mutant requires L-isoleucine can be used as microorganisms capable of producing L-threonine by fermentation (Japanese Examined Patent Publication No. 4440/1968).

Besides, the method using the mutant having a sensitivity to antibiotic borrelidin and requiring L-methionine and L-valine has been known with respect to producing L-threonine by using certain microorganism of the genus Escherichia (Japanese Examined Patent Publication No. 6752/1976).

However, there is a room for further improvement in the capability of the strains as to the amount of L-threonine accumulated and as to the yield of L-threonine from the starting materials such as glucose or fructose in the method using the microorganism above mentioned.

In DE-OS 2 044 907 a process for producing L-threonine is described. Particularily, the process is working by breeding mutants of proteins rettgeri which only needs L-isoleucine or both L-isoleucine and L-methionine for their growth and are able to grow in a medium containing $\beta$-hydroxynorvaline.

Summary of the Invention

One object of the present invention is to provide an improved process for producing L-threonine by fermentation which can give a much higher accumulated amount and yield.

Another object of the invention is to provide an improved process using a novel mutant for producing L-threonine.

These and other objects of the invention will become more apparent in the detailed description and examples hereinafter.

These objects are attained by a process for producing L-threonine by fermentation which comprises the steps of :

(a) culturing an L-threonine producing microorganism belonging to the genus Providencia or the genus Escherichia until L-threonine is accumulated in a culture broth, said microorganism having a resistance to isoleucine antagonist and

(b) recovering the accumulated L-threonine from the culture broth.

Preferred Embodiments

The microorganisms used in the present invention belong to the genus Providencia or the genus Escherichia. The genus is decided according to Bergy's Manual of Systematic Bacteriology Volume 1 (1984). Especially, the genus Providencia is decided according to pages 495 to 496 thereof. Moreover, the microorganisms used in the invention have a resistance to isoleucine antagonist and is capable to produce L-threonine.

In the invention,"isoleucine antagonist" means 1) a substance which can inhibit the growth of the microorganism belonging to the genus Providencia or the genus Escherichia, such an inhibition being reversed by supplement of isoleucine, or 2) a substance which can repress or inhibit the enzyme activity in the biosynthetic pathway of L-isoleucine.

Preferable examples of the isoleucine antagonists are thiaisoleucine, isoleucine hydroxamate, o-methylthreonine, $\beta$-methylthreonine, and so on. As for the isoleucine antagonist, thiaisoleucine is most preferably used.

The microorganisms used in the present invention include the strains which have at least the character having a resistance to isoleucine antagonist, even though these strains have other requirement for growth and a resistance to other chemical compounds.

In the invention, the microorganisms belonging to the genus Providencia preferably have a resistance to aspartic acid antagonist in addition to a resistance to isoleucine antagonist. In the invention, "aspartic acid antagonist" means (1) a substance which can inhibit the growth of the microorganism belonging to the genus Providencia, such as inhibition being reversed by supplement of aspartic acid, or (2) a substance which can repress or inhibit the enzyme activity in the biosynthetic pathway of L-aspartic acid. Preferable examples of the aspartic acid antagonist are D,L-aspartic acid hydroxamate, $\alpha$-methylaspartic acid, $\beta$-methylaspartic acid, cysteinsulfinic acid, difluorosuccinic acid, hadasizine, and so on. Above all, D,L-aspartic acid hydroxamate is most preferably used. These characters effectively operate the capability of producing L-threonine.

Moreover, in the invention there may be more preferably employed microorganisms belonging to the genus Providencia which have further characters selected from the character having a resistance to $\alpha$-amino-$\beta$-hydroxyvaleric acid, the character having an auxotrophy, which includes leaky type, for L-isoleucine or L-leucine,and the character having a resistance to feedback control by L-threonine, in addition to the characters having a resistance to isoleucine antagonist and aspartic acid antagonist. These characters also effectively operate the capability of producing L-threonine. Therefore, there may be more preferably employed microorganisms which have some or all of characters above mentioned.

Representative microorganisms useful for this invention are as follows:

(a) Providencia rettgeri TP6-28 (FERM BP-1135)

This TP6-28 strain has a resistance to thiaisoleucine, $\alpha$-amino-$\beta$-hydroxyvaleric acid and L-ethionine; and has an auxotrophy, which includes leaky type, for L-isoleucine, and requires L-leucine for the growth thereof, and was deposited with Fermentation Research Institute in Japan on July 15, 1985.

(b) Providencia rettgeri AXR 2G-10 (FERM BP-1138)

This AXR 2G-10 strain has a resistance to D,L-aspartic acid hydroxamate, thiaisoleucine, $\alpha$-amino-$\beta$-hydroxyvaleric acid and L-ethionine; and has an auxotrophy, which includes leaky type, for L-isoleucine, and requires L-leucine for the growth thereof, and was deposited with Fermentation Research Institute in Japan on May 17, 1986.

(c) Providencia rettgeri TP7-55 (FERM BP-1137)

This TP7-55 strain has a resistance to D,L-aspartic acid hydroxamate, thiaisoleucine, $\alpha$-amino-$\beta$-hydroxyvaleric acid, and L-ethionine; and has an auxotrophy, which includes leaky type, for L-isoleucine, and requires L-leucine for the growth thereof, and was deposited with Fermentation Research Institute in Japan on May 17, 1986.

(d) Escherichia coli M-5 (FERM BP-1136)

This M-5 strain has a resistance to thiaisoleucine; and requires L-methionine and L-valine for the growth thereof, and has a sensitivity to borrelidin, and was deposited with Fermentation Research Institute in Japan on February 24, 1986.

The FERM BP numbers are the deposit number of the Fermentation Research Institute Agency of Industrial Science and Technology, at No. 1-3, Yatabe-cho, Higashi 1-chome, Tsukuba-gun, Ibaragi-ken, 305 JAPAN, from which the microorganisms with the FERM BP numbers are available to any party who requests them.

These threonine producing microorganisms can be derived as a mutant, for example, from the following parent strains;

(i) Providencia rettgeri ATCC 21118

This ATCC 21118 strain requires L-isoleucine for the growth thereof and is a parent strain of Providencia rettgeri TP3-105 strain which has a resistance to $\alpha$-amino-$\beta$-hydroxyvaleric acid and L-ethionine; and has an auxotrophy, which includes leaky type, for L-isoleucine and requires L-leucine for the growth thereof. Moreover, TP3-105 strain is a parent strain of TP6-28 strain.

(ii) Providencia rettgeri AHXR 7665

This AHXR 7665 strain, which has a resistance to $\alpha$-amino-$\beta$-hydroxyvaleric acid, L-ethionine, and D,L-aspartic acid hydroxamate; and has an auxotrophy including leaky type for L-isoleucine and requires L-

EP 0 213 536 B1

leucine for the growth thereof, is a mutant derived from TP3-105 strain, and is a parent strain of TP7-55 strain.

(iii) Escherichia coli ATCC 21248

This ATCC 21248 strain requires L-methionine and L-valine for the growth thereof, and is a parent strain of Escherichia coli BS-58 strain. BS-58 strain, which requires L-methionine and L-valine; and has a sensitivity to borrelidin, is a parent strain of M-5 strain.

These mutants can be relatively easily obtained by conventional mutation methods. Namely, in the case of obtaining the isoleucine antagonist resistant mutant, the parental cells are irradiated with ultraviolet light or treated with mutagene, for example, N-methyl-N'-nitro-N-nitrosoguanidine or ethylmethane sulfonate. The resistant mutant is obtained as the strain having the capability of growth, on the agar plate containing enough high concentration of isoleucine antagonist that parent strain can not grow.

In the invention, the microorganism having a resistance to isoleucine antagonist is defined as a strain of the microorganism which has a stronger resistance to isoleucine antagonist than parent strain does, and is preferably defined as a strain of the microorganism whose growth degree is at least 50 % in the culture medium containing so high concentration of isoleucine antagonist, as to reduce the relative growth degree of parent strain to less than 40 %, based on the case in the absence of isoleucine antagonist. For example, in the case of thiaisoleucine resistant strain, the microorganism having a resistance to thiaisoleucine is defined as a strain whose growth degree in the culture medium supplemented with 5 mM of thiaisoleucine is at least 50 % based on the case in the absence of thiaisoleucine. In the invention, growth degree is shown by the relative optical density of the culture broth at 660 nm when the optical density of culture broth in none-supplement of isoleucine antagonist is defined as 100 %.

Besides, in the case of obtaining the aspartic acid antagonist-resistant mutant, in addition to isoleucine antagonist-resistant mutant, the parental cells are irradiated with ultraviolet light or treated with mutagene, for example, N-methyl-N'-nitro-N-nitrosoguanidine or ethylmethane sulfonate. The resistant mutant is obtained as the strain having the capability of growth on the agar plate containing enough high concentration of aspartic acid antagonist that parent strain can not grow.

In the invention, the microorganism having a resistance to aspartic acid antagonist is defined as a strain of the microorganism which has a stronger resistance to aspartic acid antagonist than parent strain does, and is preferably defined as a strain of the microorganism whose growth degree is at least 60 % in the culture medium containing so high concentration of aspartic acid antagonist, as to reduce the relative growth degree of parent strain to less than 30 %. In the invention, the growth degree is shown by the relative optical density of the culture broth at 660 nm when the optical density of culture broth in none-supplement of aspartic acid antagonist is defined as 100 %.

In the present invention, as a culture medium for producing L-threonine can be used a conventional medium containing carbon source, nitrogen source, inorganic ions and if necessary, other organic minor ingredients.

The preferable culture medium may contain 2 to 15 % of carbon source, for example, carbonhydrates such as glucose, fructose, hydrolysate of starch or cellulose, or molasses; organic acids such as fumalic acid, citric acid, or succinic acid; alcohols such as glycerol;

contain 0.5 to 4.0 % of nitrogen source, for example, organic ammonium salts such as ammonium acetate, urea inorganic ammonium salts such as ammonium sulfate, ammonium chloride, ammonium phosphate, or ammonium nitrate; ammonia gas; aqueous ammonia;

contain 0.001 to 0.4 % of required materials such as L-valine, L-isoleucine, or L-leucine as an organic nutrient;

and if necessary, contain 0 to 4 % of corn steep liquor, polypeptone, or yeast extract and the like as an organic minor ingredient. In addition, small amount of potassium phosphate, magnesium sulfate, ferrous sulfate 7-hydrate, and 4- to 6- hydrate of manganese sulfate, etc. may be added to culture medium as an inorganic ion.

Cultivation is carried out preferably under aerobic conditions Preferable result can be obtained by adjusting the pH of the medium to from 5 to 9 and controlling a temperature from 24 to 37 ° C during cultivation, and by shaking or stirring with aeration for from 48 to 120 hours.

The recovery of accumulated L-threonine from culture broth is carried out by conventional methods. For example, the culture broth from which the cells are removed is adjusted to pH 2 with hydrochloric acid. Then, the broth solution is passed through the strongly acidic ion exchange resin, and the adsorbant is eluted by dilute aqueous ammonia. Thereafter ammonia is evaporated from the resulting eluent, and then the resulting solution is condensed. Alcohol is added to the resultant, the crystals formed under cooling are

5

collected, and then L-threonine can be obtained.

The invention will be more clearly understood with reference to the following Experiments and Examples. However, these Experiments and Examples are intended to illustrate the invention and are not to be construed as limiting the scope of the invention.

EXPERIMENT 1

(Isolation of the thiaisoleucine-resistant mutants belonging to the genus Providencia)

The cells of Providencia rettgeri TP3-105 and the cells of Providencia rettgeri AHXR 7665 were mutagenized by N-methyl-N'-nitro-N-nitrosoguanidine treatment (300μg/ml, for 10 min, at 30 °C) according to a conventional method. The resulting cells were spread on the agar plate which has the following composition.

Medium composition for agar plate

Glucose      0.5 %
(NH$_4$)$_2$SO$_4$      0.1 %
K$_2$HPO$_4$      0.7 %
KH$_2$PO$_4$      0.3 %
MgSO$_4$•7H$_2$O      0.01 %
D,L-thiaisoleucine      0.15 %
L-leucine      0.2 %
L-valine      0.2 %

Then, after incubation for 5 to 7 days at 30 °C, large colonies formed on the plate were picked up, and thiaisoleucine resistant mutants, Providencia rettgeri TP6-28 strain and TP7-55 strain were respectively obtained.

EXPERIMENT 2

(Isolation of the thiaisoleucine resistant mutant belonging to the genus Escherichia)

The cells of Escherichia coli BS-58 were mutagenized by N-methyl-N'-nitro-N-nitrosoguanidine treatment (300 μg/ml, for 10 min, at 30 °C) according to a conventional method. The resulting cells were spread on the agar plate which has the following composition.

Medium composition for agar plate

Glucose      0.5 %
(NH$_4$)$_2$SO$_4$      0.1 %
K$_2$HSO$_4$      0.7 %
KH$_2$SO$_4$      0.3 %
MgSO$_4$•7H$_2$O      0.01 %
L-methionine      0.01%
D,L-thiaisoleucine      0.15 %
L-leucine      0.2 %
L-valine      0.2 %

Then, after incubation for 5 to 7 days at 30 °C, large colonies formed on the plate were picked up, and thiaisoleucine resistant mutant, Escherichia coli M-5 strain was obtained.

EXPERIMENT 3

(Degree of resistance of the thiaisoleucine resistant nutants belonging to the genus Providencia)

Each strain shown in Table 1 was cultivated in bouillon liquid at 30 °C for 16 hours with shaking, and the grown cells were harvested and washed with physiological saline.

The resulting cell suspension was inoculated into 5 ml of the following minimal medium containing 0 mM, 2.5 mM, 5.0 mM, and 10 mM of thiaisoleucine, respectively, and cultivated at 30 °C for 24 hours, and the growth degree of each strain was measured.

Minimal medium composition

Glucose        0.5 %
$(NH_4)_2SO_4$'''  0.1 %
$K_2HPO_4$      0.7 %
$KH_2PO_4$      0.3 %
$MgSO_4 \bullet 7H_2O$     0.01 %
L-isoleucine    0.001 %
L-leucine    0.5 %
L-valine    0.5 %

The results were shown in Table 1.

## Table 1

| Strains | Relative growth degree *)    (%) | | | |
|---|---|---|---|---|
| | Concentration of thiaisoleucine added (mM) | | | |
| | 0 | 2.5 | 5.0 | 10.0 |
| Parental strain Providencia rettgeri TP3-105 | 100 | 65.0 | 37.8 | 10.5 |
| Providencia rettgeri TP6-28 | 100 | 90.6 | 82.3 | 73.5 |
| Providencia rettgeri AXR2G-10 | 100 | 95.1 | 94.4 | 83.3 |
| Providencia rettgeri TP7-55 | 100 | 92.0 | 94.9 | 93.4 |

*) Relative growth degree is shown by the relative
optical density of the culture broth at 660 nm
when the optical density of the culture broth
in the absence of thiaisoleucine is 100 %.

The growth of the thiaisoleucine resistant mutants, Providencia rettgeri TP6-28 strain, AXR 2G-10 strain and TP7-55 strain used in the present invention, were not inhibited by thiaisoleucine compared with that of parental strain Providencia rettgeri TP3-105 strain.

Therefore, it is evident that these mutants have a resistance to thiaisoleucine.

EXPERIMENT 4

(Degree of resistance of the thiaisoleucine resistant mutant belonging to the genus Escherichia)

Each strain shown in Table 2 was cultivated in bouillon liquid at 30 °C for 16 hours with shaking, and the grown cells were harvested and well washed with physiological saline.

The resulting cell suspension was inoculated into 5 ml of the following minimal medium containing 0 mM, 5 mM, 10 mM of thiaisoleucine, respectively, and cultivated at 30 °C for 24 hours, and the growth degree of each strain was measured.

Minimal medium composition

Glucose      0.5 %
$(NH_4)_2SO_4$      0.1 %
$K_2HPO_4$      0.7 %
$KH_2PO_4$      0.3 %
$MgSO_4 \bullet 7H_2O$      0.01 %
L-methionine      0.01 %
L-valine      0.01 %

The results were shown in Table 2.

## Table 2

| | Relative growth degree *) (%) | | |
| | Concentration of thiaisoleucine added (mM) | | |
| | 0 | 5 | 10 |
| **Parental strain** | | | |
| Escherichia coli BS-58 | 100 | 0.51 | 0.34 |
| Escherichia coli M-5 | 100 | 102 | 67.9 |

*) Relative growth degree is shown by the relative optical density of the culture broth at 660 nm when the optical density of the culture broth in the absence of thiaisoleucine is 100 %.

The growth of the thiaisoleucine resistant mutant, Escherichia coli M-5 strain used in the present invention, is not inhibited by thiaisoleucine compared with that of parental strain Escherichia coli BS-58 strain. Therefore, it is evident that this mutant has a resistance to thiaisoleucine.

EXPERIMENT 5

(Isolation of the mutant strain having a resistance to D,L-aspartic acid hydroxamate)

The cells of Providencia rettgeri TP6-28 and the cells of Providencia rettgeri TP3-105 were mutagenized by N-methyl-N'-nitro-N-nitrosoguanidine treatment (300 μg/ml, for 10 min, at 30 °C) according to a conventional method. The resulting cells were spread on the agar plate which has the following composition.

Medium composition for agar plate

Glucose        0.5 %
$(NH_4)_2SO_4$        0.1 %
$K_2HPO_4$        0.7 %
$KH_2PO_4$        0.3 %
$MgSO_4 \bullet 7H_2O$        0.01 %
L-isoleucine        0.005%
L-leucine        0.005 %
D,L-aspartic acid hydroxamate        2 g/ℓ

Then, after incubation for 5 to 8 days at 30 °C, large colonies formed on the plate were picked up, and D,L-aspartic acid hydroxamate resistant mutants, Providencia rettgeri AXR 2G-10 strain and AHXR 7665 strain, were respectively obtained.

Experiment 6

(Degree of resistnace of D,L-aspartic acid hydroxamate resistant mutants)

Each strain shown in Table 3 was cultivated in bouillon liquid at 30 °C for 16 hours with shaking, and grown cells were harvested and washed with physiological saline.

The resulting cell suspension was inoculated into 5 ml of the following minimal medium containing 0 g/ℓ , 0.25 g/ℓ , 0.5 g/ℓ , 1.0 g/ℓ , 2.0 g/ℓ of D,L-aspartic acid hydroxamate, respectively, and cultivated at 30 °C for 24 hours, and the growth degree of each strain was measured.

Minimal medium composition

Glucose        0.5 %
$(NH_4)_2SO_4$        0.1 %
$K_2HPO_4$        0.7 %
$KH_2PO_4$        0.3 %
$MgSO_4 \bullet 7H_2O$        0.01 %
L-isoleucine        0.005 %
L-leucine        0.005 %

The results are shown in Table 3.

## Table 3

| | Relative grwoth degree *) (%) | | | | |
| --- | --- | --- | --- | --- | --- |
| | Amount of D,L-aspartic acid hydroxamate added (g/ℓ) | | | | |
| | 0 | 0.25 | 0.5 | 1.0 | 2.0 |
| **Parental strain** | | | | | |
| Providencia rettgeri TP3-105 | 100 | 1.9 | 0.7 | 0.4 | 0.9 |
| Providencia rettgeri AXR 2G-10 **) | 100 | 85.8 | 67.6 | 55.0 | 1.9 |
| Providencia rettgeri TP7-55 ***) | 100 | 83.4 | 68.0 | 56.0 | 38.4 |

*) Relative growth degree is shown by the relative optical density of the culture broth at 660 nm when the optical density of the culture broth in the absence of D,L-aspartic acid hydroxamate is 100 %.

**) Providencia rettgeri AXR 2G-10 strain was derived from TP6-28 strain according to Experiment 1.

***) Providencia rettgeri TP7-55 strain was derived from AHXR 7665 strain according to Experiment 5.

The growth of D,L-aspartic acid hydroxamte resistant mutants Providentcia rettgeri AXR 2G-10 strain and TP7-55 strain used in the present invention were not inhibited in the presence of high concentration of D,L-aspartic acid hydroxamate compared with that of parental strain TP3-105 strain. Therefore, it is evident that these mutant have a strong resistance to D,L-aspartic acid hydroxamate.

EXAMPLE 1

50 ml of fermentation medium consisting of the following composition in 1 ℓ conical flask was sterilized at 115 °C for 10 minutes.

Fermentation medium composition

Glucose (sterilized separately)     8%
$(NH_4)_2SO_4$     2.5 %
$KH_2PO_4$     0.1 %
$MgSO_4 \bullet 7H_2O$     0.04 %
$Fe^{++}$     2 ppm
$Mn^{++}$     2 ppm
L-isoleucine     0.005 %
L-leucine     0.08 %
$CaCO_3$ (sterilized separately)     4 %
pH     7.0 (neutralized with KOH)

Each strain shown in Table 4 was cultivated at 30 °C for 16 hours with shaking in bouillon liquid. 5 ml of the resulting each culture broth was put into the fermentation medium, and then cultivation was carried at 30 °C for 90 hours with shaking condition of 110 rpm and 5 cm-stroke.

After cultivation, the amount of L-threonine in the filtrate which was obtained by removing the cells and calcium carbonate from culture broth was quantitatively analyzed by automatic amino acid analyzer (produced by Japan Electric Co. JLC-200A) and the results shown in Table 4 were obtained.

Table 4

|  | Strains | Amount of L-threonine accumulated (g/ℓ) | Yield of *) L-threonine produced (%) |
|---|---|---|---|
| Comparative example | Providencia rettgeri TP3-105 | 12.9 | 17.2 |
| Example Example | Providencia rettgeri TP6-28 Providencia rettgeri AXR 2G-10 Providecia rettgeri TP7-55 | 20.4 23.4 22.0 | 24.8 29.2 28.0 |

*) Yield of L-threonine produced was based on the consumed glucose.

Both amount of L-threonine accumulated and yield of L-threonine produced were obviously improved in Examples, Providencia rettgeri TP6-28 strain, AXR 2G-10 strain and TP7-55 strain.

EXAMPLE 2

Each strain shown in Table 5 was cultivated in bouillon liquid medium at 30 °C for 16 hours with shaking, and this culture broth was inoculated by 10 % by volume into a small glass jar fermentor containing 800 ml of the same fermentation medium as used in Example 1 except that 0.5 % of $(NH_4)_2SO_4$ and 4.0 % of glucose were used. Cultivation with aeration (1 vvm) and agitation (800 rpm) was started at 30 °C.

Controll of pH and feed of nitrogen source were served with 25 % aqueous ammonia and pH was kept from 6.5 to 8.0. Cultivation was carried out with intermittent feeding of glucose, $KH_2PO_4$, $MgSO_4 \bullet 7H_2O$, L-leucine and L-isoleucine for 64 hours and the results shown in Table 5 were obtained.

Table 5

| | | Amount of L-threonine accumulated (g/ℓ) | Yield of L-threonine accumulated (%) | Amount of alanine accumulated (g/ℓ) | Amount of valine accumulated (g/ℓ) |
|---|---|---|---|---|---|
| Comparative example | Providencia rettgeri TP3-105 | 39.0 | 22.5 | 4.5 | 6.6 |
| Example | Providencia TP6-28 | 72.5 | 29.7 | 5.0 | 7.0 |
| | Providencia rettgeri AXR 2G-10 | 50.0 | 33.3 | 2.0 | ND* |
| | Providecrettgeri TP7-55 | 72.0 | 38.0 | 4.0 | 8.0 |

*)not detected

The cells were removed from the cultre broth of Providencia rettgeri TP6-28. 500 ml of the resulting filtrate was passed through the column packed with strong cation exchange resin DIAION (Trade Name) SK•1B (H type). Then, the column was washed with water and thereafter the adsorbant in the column was eluted by 2 N aqueous ammonia. The eluent was concentrated under reduced pressure after decolorizing.

Ethanol was added to the resultant and left standing under cooling, and then the crystals formed were collected, dried to give 32.3 g of L-threonine having over 96 % of purity.

EXAMPLE 3

After each strain shwon in Table 6 was cultivated in bouillon liquid medium at 30 °C for 16 hours with shaking. The resulting culture broth was put into 1 ℓ conical flask which was charged with 40 ml of fermentation medium consisting of the following composition, and which was sterilized at 115 °C for 10 minutes beforehand.

Fermentation medium composition

Glucose (sterilized separately)     5 %
$(NH_4)_2SO_4$     1 %
$KH_2PO_4$     0.05 %
$K_2HPO_4$     0.05 %
$MgSO_4•7H_2O$     0.05 %
$Fe(SO_4)_3•6H_2O$     0.01 %
D,L-Methionine     0.006 %
L-Valine     0.04 %
$CaCO_3$ (sterilized separately)     1 %
pH     6.8 (neutralized with KOH)

Cultivation was carried out at 30 °C for 72 hours with shaking conditions of 110 rpm and 5 cm-stroke.

After cultivation, the amout of L-threonine in the filtrate which was obtained by removing the cells and calcium carbonate from culture broth was quantitatively analyzed by automatic amino acid analyzer (produced by Japan Electric Co. JLC-200A) , and the results shown in Table 6 were obtained.

Table 6

|  | Strain | Amount of L-threonine accumulated (g/ℓ) | Yield of[*] L-threonine produced (g/ℓ) |
|---|---|---|---|
| Comparative Example | Escherichia coli BS-58 | 6.0 | 12.4 |
| Exmaple | Escherichia coli M-5 | 8.9 | 18.3 |

*) Yield of L-threonine produced was based on the consumed glucose.

Both amount of L-threonine accumulated and yield of L-threonine produced were obviously improved in Example, Escherichia coli M-5. The cells were removed from the culture broth of Escherichia coli M-5. 200 ml of the resulting filtrate was passed through the column packed with strong cation exchange resin DIAION (Trade Name) SK•1B (H type). Then, the column was washed with water and thereafter the adsorbant in the column was eluted by 2 N aqueous ammonia. The eluent was concentrated under reduced pressure after decolorizing

Ethanol was added to the resultant and left standing under cooling, and then the crystals formed were collected, dried to give 1.55 g of L-threonine having over 96 % of purity.

**Claims**

1.   A process for producing L-threonine by fermentation which comprises the steps of:
    (a) culturing an L-threonine producing microorganism belonging to the genus Providencia or the genus Escherichia until L-threonine is accumulated in a culture broth, said microorganisms having a resistance to isoleucine antigonist and
    (b) recovering the accumulated L-threonine from the culture broth.

EP 0 213 536 B1

**2.** A process according to claim 1, wherein said isoleucine antagonist is thiaisoleucine.

**3.** A process according to claim 1, wherein said microorganism belongs to the genus Providencia.

**4.** A process according to claim 3, wherein said microorganism further has a resistance to aspartic acid antagonist.

**5.** A process according to claim 4, wherein said aspartic acid antagonist is aspartic acid hydroxamate.

**6.** A process according to claim 3, wherein said microorganism further has a resistance to $\alpha$-amino-$\beta$-hydroxyvaleric acid and L-ethionine; and has an auxotrophy, which includes leaky type, for L-isoleucine, and requires L-leucine for the growth thereof.

**7.** A process according to claim 1, wherein said microorganism belongs to the genus Escherichia, and further requires L-methionine and L-valine for the growth thereof and has a sensitivity to borrelidin.

**Revendications**

**1.** Procédé de préparation de la L-thréonine par fermentation, qui comprend les stades consistant à :
(a) cultiver un microorganisme produisant de la L-thréonine, appartenant au genre Providencia ou au genre Escherichia jusqu'à ce que de la L-thréonine se soit accumulée dans le bouillon de culture, ces microorganismes résistant à l'antagoniste de l'isoleucine, et.
(b) récupérer la L-thréonine accumulée dans le bouillon de culture.

**2.** Procédé selon la revendication 1, dans lequel cet antagoniste de l'isoleucine est la thiaisoleucine.

**3.** Procédé selon la revendication 1, dans lequel ce microorganisme appartient au genre Providencia.

**4.** Procédé selon la revendication 3, dans lequel ce microorganisme résiste en outre à l'antagoniste de l'acide aspartique.

**5.** Procédé selon la revendication 4, dans lequel cet antagoniste de l'acide aspartique est l'hydroxamate de l'acide aspartique.

**6.** Procédé selon la revendication 3, dans lequel ce microorganisme présente en outre une résistance à l'acide $\alpha$-amino-$\beta$-hydroxyvalérique et à la L-éthionine; et présente une auxotrophie qui comprend le type "fuyard" pour la L-isoleucine et exige de la L-leucine pour sa croissance.

**7.** Procédé selon la revendication 1, dans lequel ce microorganisme appartient au genre Escherichia et exige en outre de la L-méthionine et de la L-valine pour sa croissance, et est sensible à la borrélidine.

**Patentansprüche**

**1.** Verfahren zur Herstellung von L-Threonin durch Fermentation, welches die Schritte umfaßt:
a) Kultivierung eines L-Threonin erzeugenden Mikroorganismus, der zur Gattung Providencia oder zur Gattung Escherichia gehört, bis sich L-Threonin in der Bouillonkultur angesammelt hat, wobei dieser Mikroorganismus eine Beständigkeit gegenüber Isoleucin-Antigonist aufweist und
b) Gewinnung des angesammelten L-Threonins aus der Bouillonkultur.

**2.** Verfahren nach Anspruch 1, worin das Isoleucin-Antagonist Thiaisoleucin ist.

**3.** Verfahren nach Anspruch 1, worin der Mikroorganismus zur Gattung Providencia gehört.

**4.** Verfahren nach Anspruch 3, worin der Mikroorganismus außerdem eine Beständigkeit gegenüber Asparaginsäure-Antagonist aufweist.

**5.** Verfahren nach Anspruch 4, worin das Asparaginsäure-Antagonist Asparaginsäurehydroxamat ist.

14

**6.** Verfahren nach Anspruch 3, worin der Mikroorganismus außerdem eine Beständigkeit gegenüber $\alpha$-Amino-$\beta$-hydroxyvaleriansäure und L-Ethionin aufweist; und eine Auxotrophy, die den durchlässigen Typ einschließt, für L-Isoleucin hat und zu seinem Wachstum L-Leucin benötigt.

**7.** Verfahren nach Anspruch 1, worin der Mikroorganismus zur Gattung Escherichia gehört und außerdem zu seinem Wachstum L-Methionin und L-Valin benötigt und eine Empfindlichkeit gegenüber Borrelidin aufweist.

15